(19) **Europäisches Patentamt / European Patent Office / Office européen des brevets**

(11) **EP 2 864 035 B1**

(12) **FASCICULE DE BREVET EUROPEEN**

(45) Date de publication et mention de la délivrance du brevet:
**24.10.2018 Bulletin 2018/43**

(51) Int Cl.:
**B01J 13/18** (2006.01)      **A61K 8/11** (2006.01)
**A61K 9/50** (2006.01)      **C09B 67/00** (2006.01)
**C11D 3/50** (2006.01)

(21) Numéro de dépôt: **13737339.5**

(22) Date de dépôt: **20.06.2013**

(86) Numéro de dépôt international:
**PCT/FR2013/051439**

(87) Numéro de publication internationale:
**WO 2013/190241 (27.12.2013 Gazette 2013/52)**

(54) **PROCEDE DE PREPARATION D'UN MATERIAU MULTICOMPARTIMENTE POUR LA DELIVRANCE THERMOSTIMULEE DE SUBSTANCES D'INTERET**

HERSTELLUNGSVERFAHREN FÜR MEHRKAMMERMATERIAL ZUR THERMISCH STIMULIERTEN FREISETZUNG VON BESTIMMTEN STOFFEN

PREPARATION METHOD OF MULTI-COMPARTMENT MATERIAL FOR THE THERMALLY STIMULATED DELIVERY OF SUBSTANCES OF INTEREST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.06.2012   FR 1255846**

(43) Date de publication de la demande:
**29.04.2015   Bulletin 2015/18**

(73) Titulaire: **Centre National de la Recherche Scientifique**
**75016 Paris (FR)**

(72) Inventeurs:
• **SCHMITT, Véronique**
**33400 Talence (FR)**
• **DEPARDIEU, Martin**
**45000 Orleans (FR)**
• **NOLLET, Maxime**
**33000 Bordeaux (FR)**
• **BACKOV, Rénal**
**33200 Bordeaux-cauderan (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**WO-A1-2011/012813      DE-A1-102008 021 005**

• **MATHIEU DESTRIBATS ET AL: "Thermostimulable Wax@SiO2 Core-Shell Particles", LANGMUIR, vol. 26, no. 3, 2 février 2010 (2010-02-02), pages 1734-1742, XP055053549, ISSN: 0743-7463, DOI: 10.1021/la902828q**
• **DATABASE WPI Week 201066 Thomson Scientific, London, GB; AN 2010-M39814 XP002692823, & CN 101 824 307 A (UNIV BEIJING CHEM TECHNOLOGY) 8 septembre 2010 (2010-09-08)**
• **DATABASE WPI Week 199514 Thomson Scientific, London, GB; AN 1995-102158 XP002692824, & JP 7 026251 A (MITSUBISHI JUKOGYO KK) 27 janvier 1995 (1995-01-27)**
• **DATABASE WPI Week 198417 Thomson Scientific, London, GB; AN 1984-103940 XP002692825, & JP 59 046124 A (NIPPON SANSO KK) 15 mars 1984 (1984-03-15)**

**Description**

La présente invention est relative à un procédé de préparation d'un matériau constitué d'une enveloppe de silice renfermant une phase aqueuse comprenant au moins une gouttelette d'huile cristallisable à l'état solide.

Il peut être utile d'encapsuler des molécules d'intérêt telles que des médicaments, des colorants, des pigments, des réactifs, des parfums, des pesticides, etc..., pour les protéger des agressions extérieures, notamment de l'oxydation, pour les acheminer vers un lieu d'administration où elles pourront être délivrées ou bien encore pour les stocker avant une utilisation dans des conditions où elles seront libérées de leur capsule sous l'influence d'un stimulus externe. Une des premières applications de la micro encapsulation été la mise au point d'un papier copiant sans carbone commercialisé à la fin des années 60 dans lequel des microcapsules emprisonnant une encre étaient présentes sur le verso d'une feuille de papier de façon à libérer l'encre par rupture des capsules sous la pression exercée par la pointe d'un stylo lors de l'écriture. De nos jours, l'encapsulation se développe dans différents secteurs industriels tels que les industries pharmaceutique, cosmétique, alimentaire, textile et agricole. Les capsules et microcapsules deviennent de plus en plus sophistiquées, notamment dans le domaine pharmaceutique où elles permettent de faire de la délivrance contrôlée et/ou ciblée de principes actifs

Différents types et morphologies de capsules et microcapsules ont déjà été proposées tels que par exemple des capsules protéiques, des cyclodextrines, des liposomes, des vésicules lamellaires concentrées, des émulsions doubles, des colloidosomes, des microcapsules à enveloppes de silice, des nanocapsules de silice et de polymères thermosensibles tel que le poly(*N*-isopropylacrylamide (PNIPAM), des microsphères d'hydrogel thermosensible, des microsphères de PNIPAM-Polylactide, etc....De nombreuses méthodes permettant de préparer ces différents type de capsules et microcapsules ont également été développées et mises au point durant ces dernières années, telles que par exemple et de façon non exhaustive la précipitation de polymères par séparation de phase, le dépôt d'électrolyte couche par couche, la polymérisation par polycondensation interfaciale, etc...

L'inconvénient des techniques déjà connues est que la libération des molécules d'intérêt à partir des capsules et microcapsules proposées dans l'art antérieur est le plus souvent lente et progressive, c'est-à-dire prolongée dans le temps.

Il a alors été proposé de remédier à ce problème en proposant des matériaux constitués d'une enveloppe de silice renfermant un coeur de cire contenant une ou plusieurs substances d'intérêt, ces matériaux étant préparés par minéralisation d'une émulsion de Pickering, c'est-à-dire d'une émulsion de type huile-dans-eau dans laquelle la dispersion des gouttelettes d'huile dans l'eau est stabilisée par des nanoparticules colloïdales adsorbées à l'interface eau/huile (voir par exemple la demande de brevet FR-A-2 948 581 ou WO2011/012813 A1). En utilisant une huile cristallisable pour préparer ces matériaux, c'est-à-dire une huile dont la température de fusion ($T_F$) est assez basse (par exemple 37°C), il est possible de préparer des matériaux dans lesquels la phase encapsulée est solide dans les conditions de stockage (par exemple à température ambiante) mais devient liquide dans les conditions d'utilisation du matériau (par exemple à une température supérieure à 37°C dans le cas d'un médicament ingérable ou injectable), provoquant ainsi la rupture de la capsule par fusion et expansion thermique de la phase encapsulée et la libération concomitante et rapide de la ou des substances actives contenues dans la phase encapsulée. Ces matériaux permettent d'encapsuler aussi bien des substances d'intérêt lipophiles (sous forme solubilisée) que des substances d'intérêt hydrophiles (sous forme dispersée). Cependant, lorsque la phase encapsulée de ces matériaux renferme plusieurs substances de nature chimique différente, celles-ci sont au contact les unes des autres ce qui ne permet pas, au sein d'un même matériau, d'encapsuler des substances pouvant présenter une incompatibilité chimique et/ou physique. De plus, ces matériaux ne permettent pas de contenir à la fois des substances hydrophiles et lipophiles toutes deux sous forme solubilisées puisque seules les substances lipophiles peuvent être présentes sous forme solubilisée dans la phase grasse encapsulée par l'enveloppe de silice alors que les substances hydrophiles y sont sous forme dispersée.

Il n'existe donc pas à ce jour de système compartimenté permettant d'encapsuler sous forme solubilisée, au sein d'un même matériau, au moins deux substances d'intérêt pouvant par ailleurs éventuellement être incompatibles entre elles, lesdites substances étant lipophiles ou hydrophiles et autorisant une libération rapide et totale de ces substances d'intérêt sous l'effet d'un stimulus extérieur en conditions douces.

Le but de la présente divulgation est donc de proposer un matériau permettant d'encapsuler plusieurs molécules d'intérêt de façon compartimentée et qui permettent également leur libération rapide et totale sous l'influence d'un stimulus extérieur, et en particulier d'une augmentation de la température.

La présente divulgation a pour objet un matériau sous forme de particules solides de diamètre variant de 1 $\mu$m à 1 cm constituées d'une enveloppe continue comprenant au moins un oxyde de silicium, ladite enveloppe emprisonnant une phase aqueuse, ledit matériau étant caractérisé en ce que ladite phase aqueuse renferme au moins substance d'intérêt hydrophile $S_H$ et au moins une gouttelette d'une phase grasse comprenant de 50 à 99,9 % en masse par rapport à la masse de ladite phase grasse d'une huile cristallisable à l'état solide à la température de stockage dudit matériau, ladite huile cristallisable ayant une température de fusion ($T_F$) inférieure à 100°C, et renfermant au moins une substance d'intérêt lipophile $S_L$.

Selon la présente invention, on entend par « température de stockage dudit matériau », la température à laquelle le matériau conforme à la présente invention est conservé avant son utilisation. Cette température est toujours inférieure au point de fusion de l'huile cristallisable contenue dans la phase grasse. La température de stockage correspond à une température inférieure ou égale à 20° C.

Le matériau conforme à la présente divulgation présente la particularité suivante : lorsque l'on soumet le matériau à une température supérieure à la température de fusion de l'huile cristallisable, on observe une expansion thermique de la phase grasse entraînant la rupture de l'enveloppe de silice emprisonnant la phase aqueuse et la libération rapide et totale de la phase aqueuse comprenant la ou les substances d'intérêt hydrophiles $S_H$ ainsi que de la phase grasse en fusion (c'est-à-dire à l'état liquide) comprenant la ou les substances d'intérêt lipophiles $S_L$. Ce résultat est tout à fait surprenant dans la mesure où l'oxyde de silicium entrant dans la constitution de l'enveloppe est connu pour être un isolant thermique. De plus, il n'était pas du tout évident que l'expansion thermique de la phase grasse contenue dans la phase aqueuse permette d'entrainer la rupture de l'enveloppe de silice encapsulant la phase aqueuse.

On entend dans le cadre de cet exposé par le terme « huile cristallisable », les matières grasses et les mélanges de matières grasses, d'origine naturelle (animale ou végétable) ou synthétique, dont le point de fusion est supérieur à 15°C, de préférence dont le point de fusion varie de 20 à 100°C environ, et en particulier de 20 à 50°C environ. Tous les points de fusion mentionnés dans la description de la présente demande font référence à des points de fusion déterminés par calorimétrie différentielle à balayage à pression atmosphérique ou « *Differential Scanning Calorimetry* » (DSC) en anglais.

L'huile cristallisable forme une partie majoritaire de la phase grasse et peut même, outre la ou les substances d'intérêt $S_L$ qu'elle renferme, être l'unique constituant de celle-ci. L'huile cristallisable représente de préférence de 75 à 99,9 % en masse environ par rapport à la masse de la phase grasse.

Le choix de l'huile cristallisable dépend naturellement de l'application envisagée pour le matériau et donc de la température à laquelle on souhaite observer l'expansion thermique de la phase grasse et par voie de conséquence la rupture de l'enveloppe de silice encapsulant la phase aqueuse. Parmi les huiles cristallisables utilisables selon l'invention, on peut notamment citer les paraffines telles que les paraffines ayant un point de fusion entre 42 et 44°C ou entre 46 et 48°C [RN-8002-74-2] vendues par la société Merck, ; les triglycérides ; les acides gras ; les colophanes ; les cires (alcanes longs) tels que l'eicosane et l'octadécane ; les huiles végétales hydrogénées ainsi que leurs mélanges ; et les bitumes synthétiques. Ces huiles peuvent être utilisées seules ou en mélanges.

Le matériau conforme à la présente divulgation se présente de préférence sous la forme d'une poudre de particules sphériques ou sensiblement sphériques.

Le diamètre des particules varie de préférence de 5 $\mu$m à 500 $\mu$m environ, et encore plus préférentiellement de 10 à 200 $\mu$m.

Le diamètre de la ou des gouttelettes de phase grasse présente(s) dans chaque particule du matériau conforme à la divulgation varie généralement de 8 à 80 $\mu$m et de préférence de 30 $\mu$m à 70$\mu$m.

Selon une forme de réalisation préférée de l'invention, chaque particule de matériau conforme à la divulgation ne comprend qu'une seule gouttelette de phase grasse dans la phase aqueuse contenue dans l'enveloppe de silice et le volume de ladite gouttelette de phase grasse représente de 30 à 70 % du volume interne des particules.

L'enveloppe de silice doit avoir une épaisseur suffisante pour avoir une résistance mécanique permettant l'encapsulation de la phase aqueuse. Cependant, elle doit également présenter une épaisseur lui permettant de se rompre lors d'une élévation de la température à une température supérieure à la température de fusion de la phase grasse présente dans la phase aqueuse. L'épaisseur de l'enveloppe de silice varie généralement de 0,1 à 2 $\mu$m environ, et préférentiellement de 0,2 à 2 $\mu$m environ.

En plus de l'oxyde de silicium, l'enveloppe de silice peuvent en outre comprendre un ou plusieurs oxydes métalliques de formule $MeO_2$ dans laquelle Me est un métal choisi parmi Zr, Ti, Th, Nb, Ta, V, W et Al. Dans ce cas, l'enveloppe de silice est constituée d'une matrice mixte de type $SiO_2$-$MeO_2$ dans laquelle la teneur en $MeO_2$ reste minoritaire par rapport à la teneur en oxyde de silicium, préférentiellement la teneur en $MeO_2$ représente de 1% à 40% massique, plus particulièrement de 5% à 30% massique par rapport à la masse totale de l'enveloppe.

Parmi les substances d'intérêt pouvant être incorporées dans la phase aqueuse et dans la phase grasse contenue dans la phase aqueuse du matériau conforme à la présente invention, on peut notamment citer les médicaments (principes actifs), les principes actifs utilisables en cosmétique, les réactifs chimiques, les colorants, les pigments, les encres, etc ... Bien entendu, ces substances sont incorporées dans la phase grasse présente dans la phase aqueuse lorsqu'elles sont lipophiles et dans la phase aqueuse lorsqu'elles sont hydrophiles, l'homme du métier sachant discriminer le caractère lipophile ou hydrophile d'une substance donnée en fonction de sa valeur HLB (« *Hydrophilic-Lipophilic Balance* » : balance hydrophile/hydrophobe).

A titre d'exemples de médicaments, on peut mentionner les bactéricides tels que les antiseptiques et les antibiotiques, les antis inflammatoires, les analgésiques, les laxatifs locaux, les hormones, les protéines, etc...

A titre d'exemples de principes actifs cosmétiques, on peut notamment citer les vitamines, les filtres solaires, les antioxydants tels que les antiradicalaires comme la superoxyde dismutase, les parfums, les agents absorbeurs d'odeur, les agents déodorants, les agents anti transpirants, les colorants, les pigments, les émollients, les agents hydratants, etc...

A titre d'exemples de réactifs chimiques, on peut notamment citer, les réactifs colorés, les indicateurs colorés tels que les indicateurs de pH, les catalyseurs, les amorceurs de polymérisation, les monomères, les complexants, etc...

La ou les substances d'intérêt $S_L$ représentent généralement de 0,001 à 50 % en masse environ, et préférentiellement de 0,01 à 25% en masse environ de la masse de la phase grasse contenue dans la phase aqueuse.

La ou les substances d'intérêt $S_H$ représentent généralement de 0,1 à 50 % en masse environ, et préférentiellement de 0,1 à 25% en masse environ de la masse de la phase aqueuse.

La phase aqueuse et/ou la phase grasse contenue dans la phase aqueuse peuvent en outre renfermer un ou plusieurs additifs classiquement utilisés dans les émulsions et parmi lesquels on peut notamment mentionner à titre d'exemple les protecteurs ou de conservation de la substance d'intérêt, tels que les antioxydants, les agents anti-UV, etc....

L'invention a pour objet un procédé de préparation du matériau tel que défini ci-dessus. Ce procédé est caractérisé en ce qu'il comporte les étapes suivantes consistant :

1) dans une première étape, à porter une première phase grasse PG1 comprenant de 50 à 99,9 % en masse par rapport à la masse de ladite phase grasse PG1 d'une huile cristallisable (HC) solide ayant une température de fusion $T_F$ inférieure à 100°C à une température $T_{HC}$ telle que $T_{Hc}$ est supérieure à $T_F$, pour obtenir une phase grasse PG1 à l'état liquide ;

2) dans une deuxième étape, à incorporer à la phase grasse PG1 à l'état liquide au moins une substance d'intérêt lipophile ($S_L$) ;

3) dans une troisième étape, à mettre en contact ladite phase grasse PG1 à l'état liquide avec une phase aqueuse (PA) préalablement portée à une température $T_{PA}$ telle que $T_{PA}$ est supérieure à $T_F$, ladite phase aqueuse renfermant en outre au moins une substance d'intérêt hydrophile ($S_H$) et des particules solides colloïdales ;

4) dans une quatrième étape, et toujours à une température supérieure à $T_F$, à soumettre le mélange liquide résultant de la troisième étape à une agitation mécanique pour obtenir une émulsion huile-dans-eau (H/E) formée de gouttelettes de phase grasse PG1 à l'état liquide dispersées dans la phase aqueuse continue et dans laquelle les particules solides colloïdales sont présentes à l'interface formée entre la phase aqueuse continue et les gouttelettes de phase grasse PG1 dispersées ;

5) dans une cinquième étape, et toujours à une température supérieure à $T_F$, à ajuster le pH de l'émulsion H/E obtenue ci-dessus à l'étape précédente à une valeur inférieure ou égale à 1 à l'aide d'un agent acidifiant ;

6) dans une sixième étape, et toujours à une température supérieure à $T_F$, à ajouter l'émulsion H/E obtenue ci-dessus à l'étape précédente, à une phase grasse PG2 comprenant une huile liquide à la température $T_{H/E}$ et renfermant en outre au moins un agent tensioactif non ionique et au moins un précurseur d'oxyde de silicium ;

7) dans une septième étape, et toujours à une température supérieure à $T_F$, à soumettre le mélange résultant de la sixième étape à une agitation mécanique pour obtenir une émulsion double huile-dans-eau-dans-huile (H/E/H) formée d'une phase huileuse continue (PG2) renfermant des gouttelettes de phase aqueuse PA, chacune desdites gouttelettes de phase aqueuse renfermant au moins une gouttelette de phase grasse PG1 à l'état solide ;

8) dans une huitième étape, à laisser revenir l'émulsion double H/E/H obtenue ci-dessus à l'étape précédente jusqu'à une température $T_{H/E/H}$ inférieure à $T_F$, sans agitation, afin de provoquer d'une part la solidification de la phase grasse PG1, et la formation d'une enveloppe d'oxyde de silicium autour desdites gouttelettes de phase aqueuse PA (minéralisation de l'émulsion) et ainsi obtenir le matériau attendu ;

9) dans une neuvième étape, à séparer ledit matériau de la phase grasse PG2.

L'huile cristallisable utilisée lors de la première étape, ainsi que les substances d'intérêt lipophile et hydrophile mentionnées respectivement aux étapes 2) et 3) sont telles que définies précédemment en référence au matériau conforme à l'invention.

Les particules solides colloïdales présentes dans la phase aqueuse utilisée lors de la troisième étape peuvent être minérales ou organiques. De préférence, il s'agit de particules minérales choisies dans le groupe des oxydes, hydroxydes et sulfates de métaux. Parmi de tels oxydes, on peut tout particulièrement citer les oxydes de silicium, de titane, de zirconium et, de fer, ainsi que leurs sels tels que les silicates (par exemple les argiles). Enfin, on peut citer les particules colloïdales de carbone. Parmi les particules solides colloïdales organiques, on peut citer notamment les particules polymériques, par exemple des particules de latex.

Afin d'être colloïdales, les particules solides présentent généralement une taille inférieure à quelques micromètres. Ainsi ces particules présentent généralement une taille moyenne comprise entre 5 et 5000 nm, et de préférence entre 5 et 500 nm.

Selon une forme de réalisation particulièrement préférée de l'invention, les particules solides colloïdales sont choisies parmi les nanoparticules d'oxyde de silicium. A titre d'exemple, on peut notamment citer les produits vendus sous la dénomination commerciale Aerosil ® par la société Evonik Degussa.

La quantité de particules solides colloïdales varie généralement de 0,01 % à 10%, en particulier de 0,1% à 7%, et de préférence de 1 à 5 % en masse environ par rapport à la masse totale de la phase aqueuse PA.

Avantageusement, la quantité de particules solides colloïdales présentes dans la phase aqueuse varie en fonction de la taille moyenne en volume des gouttelettes de phase grasse PG1 souhaitées dans l'émulsion et dont le diamètre moyen varie de 10 à 100 $\mu$m, de préférence de 10 à 30 $\mu$m, et encore plus préférentiellement de 15 à 25 $\mu$m environ.

Par ailleurs, les particules solides colloïdales présentent généralement une surface hydrophile et chargée, ce qui ne favorise pas leur adsorption à la surface des gouttelettes de la phase grasse PG1.

Ainsi, et selon une forme de réalisation préférée de l'invention, les particules solides colloïdales sont fonctionnalisées en surface pour favoriser leur adsorption à l'interface formée entre la phase grasse PG1 et la phase aqueuse PA continue lors de l'étape 4).

Les particules solides colloïdales peuvent ainsi être fonctionnalisées par des composés liés à leur surface par des liaisons covalentes. Cela peut être réalisé par traitement préalable des particules, en particulier par greffage chimique d'un composé comportant des groupements hydrophobes tel qu'un trialkoxysilane de formule R-Si-(OR')$_3$, dans laquelle R est un alkyle linéaire ou ramifié en C$_1$ à C$_{12}$, en particulier de C$_2$ à C$_{10}$, tout particulièrement n-octyle, portant éventuellement un groupe amino et R', identique ou différent de R, est un groupe alkyle linéaire ou ramifié en C$_1$ à C$_{12}$, en particulier de C$_1$ à C$_6$, et tout particulièrement éthyle.

Les particules solides colloïdales peuvent également être fonctionnalisées par adsorption de molécules de tensioactif à leur surface qui permettent de leur conférer une certaine hydrophobie, l'extrémité hydrophile du tensioactif étant adsorbée sur la surface des particules. Les tensioactifs utilisables pour fonctionnaliser les particules sont de préférence des tensioactifs cationiques ou anioniques.

Parmi ces tensioactifs, on préfère en particulier les alkylsulfates de sodium tels qu'en particulier le dodécylsulfate de sodium (SDS) et les bromures d'alkyltriméthylammonium.

Le tensioactif est de préférence choisi parmi les tensioactifs de charge opposée à celle de la surface des particules solides colloïdales. Ce choix permet de favoriser l'adsorption du tensioactif à la surface des particules.

A titre d'exemple de particules fonctionnalisées par un tensioactif, on peut notamment citer les nanoparticules de silice dont la surface est fonctionnalisée par un ammonium quaternaire telles que celles vendues sous la dénomination Aerosil® A380 par la société Evonik Degussa, de diamètre 7 nm, et dont la surface est fonctionnalisée par le bromure de cétyl-triméthylammonium (CTAB).

La fonctionnalisation des particules solides colloïdales par un tensioactif peut également être réalisée *in situ*, c'est-à-dire lors de leur introduction dans la phase aqueuse PA de l'émulsion. Dans ce cas, la phase aqueuse PA de l'émulsion renferme en outre ledit tensioactif en une quantité préférentiellement inférieure à la concentration micellaire critique (CMC), celui-ci venant alors s'adsorber à la surface des particules lorsque celles-ci sont dans la phase aqueuse de l'émulsion. De préférence, la quantité de tensioactif varie de 1/200ème à 1/2 de la CMC.

La phase aqueuse comprend principalement de l'eau et éventuellement un alcool, tel que le méthanol, l'éthanol, l'isopropanol, ou le butanol, de préférence l'éthanol.

Selon une forme de réalisation préférée de l'invention, la quantité de phase grasse PG1 utilisée lors de la troisième représente au plus 40 % en masse environ, et encore plus préférentiellement de 30 à 40 % en masse environ par rapport à la masse de la phase aqueuse.

Les agitations mécaniques réalisées lors des quatrième et septième étapes peuvent notamment être réalisée dans un dispositif destiné à émulsionner tel que par exemple dans des dispositifs vendus sous les dénominations commerciales Ultra-Turrax® ou Rayneri®.

La distribution de taille des gouttelettes de phase grasse PG1 dans l'émulsion H/E est généralement étroite (U<40%).

Le pH de la phase aqueuse lors de la cinquième étape est de préférence ajusté à une valeur variant de -0,5 à 0,5, et encore plus préférentiellement de -0,25 à 0.

L'agent acidifiant utilisé pour ajuster le pH de la phase aqueuse peut être choisi parmi les acides minéraux et organiques parmi lesquels on peut en particulier citer l'acide chlorhydrique, l'acide acétique, l'acide nitrique et l'acide sulfurique.

L'huile liquide à la température T$_{H/E}$ utilisée à titre de phase grasse PG2 lors de la sixième étape peut par exemple être choisie parmi le polydiméthylsiloxane, l'huile d'arachide, l'huile de tournesol, les triesters, etc et leurs mélanges.

L'homme du métier veillera à choisir de préférence une huile liquide à la température T$_{H/E}$ dont l'utilisation soit compatible avec le procédé conforme à l'invention, c'est-à-dire une huile liquide dans laquelle l'huile cristallisable utilisée à titre de phase grasse PG1 ne soit pas soluble.

Le tensioactif nonionique également utilisé lors de la sixième étape permet de stabiliser l'émulsion double, c'est-à-dire la dispersion des gouttelettes de phase aqueuse au sein de la phase grasse PG2. La nature du tensioactif nonionique n'est pas critique à partir du moment ou il est soluble dans la phase grasse PG2. Selon une forme de réalisation préférée de l'invention, le tensioactif nonionique est choisi parmi les polydiméthylsiloxanes cycliques, les copolymères blocs éthoxylés, les nonylphénols, et leurs mélanges.

Egalement selon une forme de réalisation préférée de l'invention, le tensioactif nonionique représente de 1 à 5% en masse environ, et encore plus préférentiellement de 2% à 4% en masse environ, par rapport à la masse totale de la phase grasse PG2.

Selon une forme de réalisation préférée de l'invention, lors de la sixième étape, l'émulsion H/E représente au maximum 20 % en masse, et encore plus préférentiellement au maximum 10 % en masse environ de la masse de la phase grasse PG2.

Les précurseurs d'oxyde de silicium utilisés lors de la sixième étape peuvent être choisis parmi les alcoxydes de silicium et en particulier parmi le tetraméthoxyorthosilane (TMOS), le tetraéthoxyorthosilane (TEOS), le diméthyldiéthoxysilane (DMDES), le (3-mercaptopropyl)triméthoxysilane, le (3-aminopropyl)triéthoxysilane, le N-(3-triméthoxysilylpropyl)pyrrole, le 3-(2,4-dinitrophénylamino)-propyltriéthoxysilane, le N-(2-aminoéthyl)-3-aminopropyl-triméthoxysilane, le phényltriéthoxysilane, le méthyltriéthoxysilane et leurs mélanges. Parmi ces précurseurs, le TEOS est particulièrement préféré. Ces précurseurs peuvent être substitués, de manière totale ou partielle, par des sols de silicate.

L'épaisseur de l'enveloppe formée autour de chaque gouttelette de phase aqueuse dépend de la quantité de précurseurs d'oxyde de silicium utilisée lors de la sixième étape et du diamètre des gouttelettes de la phase aqueuse dispersée dans la phase grasse PG2.

Selon une forme de réalisation préférée de l'invention, la quantité de précurseur d'oxyde de silicium varie de 1 à 7% en masse environ, plus particulièrement de 3 à 5% en masse environ par rapport à la masse totale de l'émulsion double.

Pour atteindre les plus grandes épaisseurs d'enveloppes, il est possible d'ajouter des quantité supplémentaires de précurseur d'oxyde de silicium pendant le déroulement de l'étape 8).

Lorsque les enveloppes de silice du matériau conforme à l'invention comprennent, outre l'oxyde de silicium, un oxyde métallique, on ajoute alors également à la phase aqueuse de l'émulsion double au moins un précurseur d'un oxyde métallique de formule $MeO_2$, ledit précurseur étant choisi parmi les alcoxydes, les chlorures ou les nitrates des métaux choisis parmi Zr, Ti, Th, Nb, Ta, V, W et Al.

Lorsqu'ils sont utilisés, la quantité de ces précurseurs d'oxyde métallique de formule $MeO_2$ varie de 1% à 7% environ, plus particulièrement de 3% à 5% en masse environ par rapport à la masse totale de l'émulsion double.

Lors de la huitième étape (minéralisation de l'émulsion), les conditions de pH acide provoquent l'hydrolyse et la condensation du ou des précurseurs d'oxyde de silicium à l'interface des gouttelettes de phase aqueuse dispersées dans la phase grasse PG2. Il y a donc formation d'une enveloppe de silice autour de chacune des gouttelettes de phase aqueuse PA contenue dans la phase grasse PG2.

Lors de la neuvième étape, le matériau conforme à l'invention peut être séparé de la phase grasse PG2 et récupéré par toute technique classique de séparation connue de l'homme du métier, telle que la filtration, la centrifugation et l'utilisation de tamis. Il est ensuite de préférence lavé, par exemple à l'eau ou à l'aide de l'huile utilisée dans la phase grasse PG2 lors de sa préparation, puis séché par exemple par lyophilisation pour donner une poudre.

Le matériau ainsi obtenu est stable au stockage pendant plusieurs mois à condition que la température de stockage soit inférieure à la température $T_F$ de la phase grasse PG1 contenue dans la phase aqueuse emprisonnée dans l'enveloppe de silice.

Le matériau conforme à la divulgation peut être utilisé sous forme de poudre ou de dispersion dans un solvant pour délivrer la ou les substances d'intérêt lipophile(s) présente(s) dans la phase grasse solide PG1 contenue dans la phase aqueuse emprisonnée dans l'enveloppe à base de silice, ainsi que la ou les substances d'intérêt hydrophile(s) présente(s) dans la phase aqueuse.

La divulgation a donc également pour objet l'utilisation d'un matériau conforme à la divulgation et tel que décrit précédemment pour la délivrance thermostimulée et simultanée d'au moins une substance d'intérêt lipophile et d'au moins une substance d'intérêt hydrophile.

La délivrance des substances d'intérêt lipophile et hydrophile est obtenue par expansion thermique de la phase grasse PG1 contenue dans la phase aqueuse induisant la rupture de l'enveloppe de silice entourant la phase aqueuse, sous l'effet d'une élévation de la température du matériau à une température de délivrance $T_D$ telle que $T_D > T_F$.

A titre d'exemple, et lorsque les substances d'intérêt sont des principes actifs lipophile et hydrophile à action médicamenteuse ou des ingrédients actifs de compléments alimentaires tels que par exemples des vitamines lipophile et hydrophile, l'huile cristallisable présente dans la phase grasse est de préférence choisie parmi les huiles cristallisables ayant un point de fusion inférieur à 37C°. Ainsi, lorsque ledit matériau est incorporé dans une composition pharmaceutique et que cette composition est administrée à un patient, par exemple par voie orale, la composition ingérée va se trouver à la température du corps, en général 37°C ou plus, ce qui va entraîner la fusion de la phase grasse et son expansion volumique et ainsi la rupture de l'enveloppe de silice entourant la phase aqueuse et la délivrance des principes actifs ou des ingrédients actifs.

Selon un autre exemple, les substances d'intérêt lipophile et hydrophile sont des principes actifs cosmétique et le matériau fait partie des composants d'une composition cosmétique à application topique, telle qu'une poudre, une crème ou un gel. L'échauffement de la phase grasse du matériau à une température supérieure à $T_F$ peut dans ce cas être provoqué par un frottement local lors de l'étalement de la composition cosmétique, ce qui induit un échauffement local entraînant la rupture des enveloppes de silice et la libération locale des substances d'intérêt. Si la composition cosmétique se présente sous la forme d'une poudre, son application par étalement peut s'accompagner d'un changement de texture (transformation de la poudre en une composition ayant un toucher gras dû à la rupture de l'enveloppe).

A titre d'autres exemples d'utilisation du matériau conforme à la divulgation, on peut notamment citer :

- l'utilisation pour la délivrance simultanée d'amorceurs lipophile et hydrophile d'une réaction de polymérisation induite par élévation de la température ; dans ce cas, le matériau conforme à l'invention peut par exemple être utilisé pour la fabrication de mousses solides dans le domaine des matériaux isolants.

La divulgation a également pour objet l'utilisation du matériau tel que décrit ci-dessus, à titre d'ingrédient, pour la préparation de produits pharmaceutiques, cosmétiques ou alimentaires, ainsi que les produits pharmaceutiques, cosmétiques ou alimentaires, renfermant, à titre d'ingrédient, au moins un matériau conforme à l'invention.

Ces compositions peuvent renfermer les supports pharmaceutiques, cosmétiques ou alimentaires classiques et bien connus de l'homme du métier, ainsi qu'un ou plusieurs tensioactifs destinés à favoriser la libération de la phase grasse liquide et de la phase aqueuse encapsulée lors de la rupture de l'enveloppe de silice.

La présente invention est illustrée par les exemples de réalisation suivants, auxquels elle n'est cependant pas limitée.

**EXEMPLES**

Les matières premières utilisées dans les exemples qui suivent sont listées ci-après :

- Paraffine 42-44 en bloc ayant une plage de fusion de 42 à 44°C (CAS n°8002-74-2), vendue par la société Merck ; l'expansion volumique de cette phase grasse en faisant passer sa température de la température ambiante à 55°C est d'environ 13 % ;
- Tetraéthoxyorthosilane pur à plus de 99 % (TEOS), Rhodamine B : société Sigma-Aldrich ;
- Bromure de cétyl-triméthylammonium (CTAB) : société ChemPur ;
- Polydiméthylsiloxanes cycliques vendus sous la dénomination commerciale DC3225C par la société Dow Corning (tensioactif nonionique) ;
- Nanoparticules de silice de 7 nm de diamètre, vendues sous la dénomination Aerosil® A380 : société Evonik Degussa ;
- Acide chlorhydrique à 37 % en volume (Carlo Erba Reagents) ;
- PDMS DC200, viscosité 200 cSt : Aldrich.

Ces matières premières ont été utilisées telles que reçues des fabricants, sans purification supplémentaire.

La concentration micellaire critique (CMC) du CTAB dans l'eau pure à température ambiante est de 0,92 mM.

Les matériaux obtenus ont été caractérisés à l'aide d'un microscope optique inversé vendu sous la dénomination commerciale Axiovert® X100 par la société Zeiss et équipé d'une platine chauffante de la société Mettler permettant de contrôler la température ainsi que les vitesses de chauffage et de refroidissement.

La distribution de taille des émulsions a été étudiée à l'aide d'un granulomètre vendu sous la dénomination commerciale Mastersizer Hydro MS2000 par la société Malvern Instrument. Les mesures granulométriques ont été faites à 25°C dans l'eau pure. L'intensité de la diffusion en fonction de l'angle qui a été collectée a été transformée en utilisant la théorie de Mie-Lorenz. La distribution de la taille des particules a été exprimée par leur diamètre moyen pondéré (D) et leur polydispersité (P) en appliquant les équations (1) et (2) suivantes :

$$D = \frac{\sum_i N_i D_i^3}{\sum_i N_i D_i^2} \qquad (1)$$

et

$$P = \frac{1}{\overline{D}} \frac{\sum_i N_i D_i^3 |\overline{D} - D_i|}{\sum_i N_i D_i^3} \qquad (2)$$

dans lesquelles :

- $D_i$ est le diamètre des particules,
- $N_i$ est le nombre total de gouttelettes de diamètre $D_i$,
- $\overline{D}$ est le diamètre médian, c'est-à-dire l'ouverture théorique du tamis telle que 50 % des particules, en masse, ont un diamètre supérieur et 50 % un diamètre inférieur.

Ces formules sont appliquées dans les granulomètres de la société Malvern Instrument.

**Exemple 1 : Préparation, caractérisations et étude d'un matériau conforme à l'invention**

Dans cet exemple on illustre la préparation, la caractérisation et l'étude d'un matériau conforme à l'invention, constitué d'une enveloppe de silice renfermant une phase aqueuse comprenant une phase grasse sous la forme d'une gouttelette d'huile cristallisable.

Il est à noter que dans cet exemple, les phases grasse et aqueuse ne renferment pas de substances d'intérêt, cet exemple étant donné pour démontrer la faisabilité structurelle du matériau compartimenté selon le procédé conforme à l'invention.

Il est facile d'extrapoler le procédé ci-après à des phases grasse et aqueuse renfermant respectivement au moins une substance d'intérêt lipophile et au moins une substance d'intérêt hydrophile, leur utilisation ne modifiant en aucune manière le procédé conforme à l'invention ni la structure du matériau obtenu.

1) Préparation du matériau

i) : Fonctionnalisation des particules de silice

72 mg de nanoparticules de silice Aerosil ® A380 ont été dispersés dans 7 ml d'eau distillée, à l'aide d'une cuve à ultra sons. On a ensuite ajouté à cette dispersion, 0,66 mg de CTAB/g de particules, cette quantité représentant environ 1/5 de la concentration micellaire critique (CMC = 0,92 mM). La surface des nanoparticules de silice étant chargée négativement, le CTAB (tensioactif cationique) vient s'adsorber à la surface des particules de silice et permet ainsi de leur conférer un caractère amphiphile. On a obtenu une phase aqueuse renfermant une dispersion de nanoparticules de silice fonctionnalisées en surface.

La quantité de CTAB a été adaptée à la masse des particules de silice afin d'obtenir une couverture spécifique de 25 nm2/molécule de CTAB à l'interface silice/eau en considérant que tout le CTAB utilisé est adsorbé à la surface des particules de silice.

ii) Préparation de l'émulsion H/E

3 g de paraffine ont été ajoutés dans un récipient contenant 7 g d'eau renfermant 72 mg de particules de silice telles que fonctionnalisées ci-dessus en i).

Le récipient a été porté à une température de 60°C afin de provoquer la fusion de la paraffine (phase grasse).

L'émulsification de la phase grasse et de la phase aqueuse a été réalisée à l'aide d'un agitateur vendu sous la dénomination Ultra-Turrax ® T25 par la société Janke & Kunkel, équipé d'un outil de dispersion S25 KV-25F, à 9000 tours/min. pendant 30 secondes. L'émulsion résultante H/E monodisperse ainsi obtenue (taille moyenne des gouttelettes de phase grasse centrée à 20 $\mu$m de diamètre a été maintenue à 60°C dans un bain thermostaté sans agitation afin de laisser le phénomène de coalescence limité se produire (adsorption des particules de silice à la surface des gouttelettes de phase grasse dispersée, ce qui permet d'améliorer l'homogénéité de la distribution de la taille des gouttelettes d'eau dispersée dans l'huile).

iii) Préparation de l'émulsion H/E/H

Le pH de la phase aqueuse continue de l'émulsion H/E obtenue ci-dessus à l'étape précédente a ensuite été ajusté à une valeur proche de 0 par ajout d'acide chlorhydrique. Cette très basse valeur de pH permet ensuite de catalyser l'hydrolyse du TEOS et sa condensation sous forme d'oxyde de silicium à l'interface PG2/PA.

0,8 g de l'émulsion H/E ainsi acidifiée ont ensuite été ajoutés à une phase grasse PG2 contenant de 8,2 g de PDMS, 0,3 g de tensioactif DC3225C et 0,5 g de TEOS.

L'émulsification de l'émulsion H/E dans la phase grasse PG2 a été réalisée à l'aide du même agitateur que ci-dessus à l'étape ii) en finissant par une agitation à 3500 tours/min. pendant 10 secondes. L'émulsion double H/E/H ainsi obtenue a ensuite été conservée à température ambiante sans agitation pendant 24 heures afin de provoquer la solidification de la phase grasse et laisser l'hydrolyse TEOS et sa condensation sous forme d'une enveloppe de silice se produire autour des gouttelettes de phase aqueuse.

Au bout de 24 heures, le matériau obtenu et sédimenté au fond du récipient a été récupéré, dispersé à nouveau dans du PDMS afin d'éliminer tout résidu d'huile cristallisable non encapsulée et toute gouttelette de phase aqueuse également non encapsulée ou ne contenant pas de phase grasse encapsulée.

2) Résultats et caractérisations

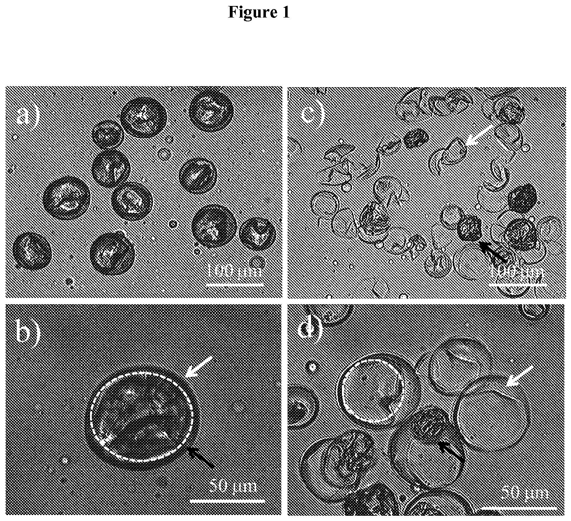

La figure 1 annexée représente une photographie en microscopie optique des particules du matériau final ainsi obtenu : la figure la est une vue de plusieurs particules après sédimentation, la figure 1b est centrée sur une seule particule et les figures 1c et 1d montrent les particules du matériau après application d'une pression mécanique à l'aide d'une spatule à température ambiante sur la lame recouvrant la goutte observée au microscope cette pression mécanique ayant entrainé la rupture des enveloppes de silice. Sur ces figures, les flèches blanches pointent les enveloppes de silice, les flèches noires pointent les gouttelettes de phase grasse solide après rupture de l'enveloppe et les cercles blancs en traits pointillés montrent que le volume réservé à la phase grasse est rempli d'une phase grasse sur la figure 1b alors qu'il est vide de phase grasse après rupture de l'enveloppe de silice sur la figure 1d.

Il a ensuite été vérifié qu'une augmentation de la température engendrait la rupture des enveloppes de silice et la libération de la phase aqueuse et de la phase grasse en fusion.

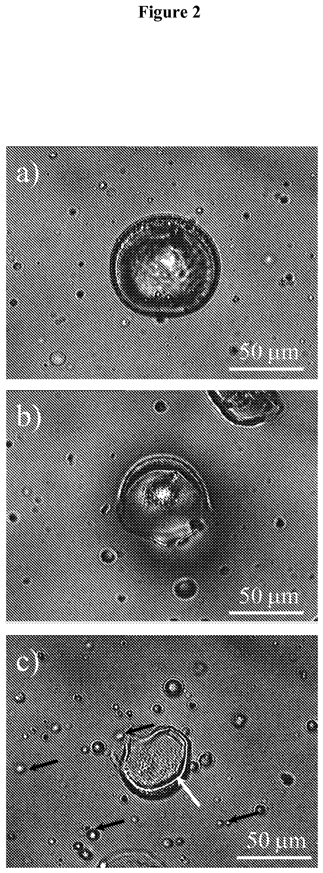

Le matériau a été incorporée à une phase huileuse constitué de PDMS et renfermant 20 % en masse de DC3225C. La composition obtenue a été portée à une température de 44°C en élevant progressivement la température à raison de 5°C par minute sur la platine chauffante du microscope. La composition a été observée en microscopie confoncale avant et après élévation de la température. Les photos correspondantes sont données sur la figure 2 annexée. Sur cette figure, la figure 2a correspond à l'image prise avant élévation de la température, alors que les figures 2b et 2c correspondent à des images prises après élévation de la température. On constate que l'élévation de la température provoque la fusion de la phase grasse contenue dans la phase aqueuse, la rupture de l'enveloppe de silice (flèche blanche sur la figure 2c) et l'expulsion de la phase grasse en fusion dans la phase huileuse (PDMS) de la composition où elle se dissous, ainsi que l'expulsion de la phase aqueuse sous la forme de quelques gouttelettes (flèches noires).

Il est ainsi possible de libérer simultanément des substances lipophiles et hydrophiles qui seraient respectivement contenues dans la phase grasse et dans la phase aqueuse PA par simple élévation de la température des matériaux conformes à l'invention.

**Revendications**

1. Procédé de préparation d'un matériau sous forme de particules solides de diamètre variant de 1 $\mu$m à 1 cm constituées d'une enveloppe continue comprenant au moins un oxyde de silicium, ladite enveloppe emprisonnant une phase aqueuse, ladite phase aqueuse renfermant au moins une substance d'intérêt hydrophile $S_H$ et au moins une gouttelette d'une phase grasse comprenant de 50 à 99,9 % en masse par rapport à la masse de ladite phase grasse d'une huile cristallisable à l'état solide à une température inférieure ou égale à 20°C, ladite huile cristallisable ayant une température de fusion $T_F$ inférieure à 100°C, et renfermant au moins une substance d'intérêt lipophile $S_L$, **caractérisé en ce que** ledit procédé comporte les étapes suivantes consistant :

   1) dans une première étape, à porter une première phase grasse PG1 comprenant de 50 à 99,9% en masse par rapport à la masse de ladite phase grasse PG1 d'une huile cristallisable HC solide ayant une température de fusion $T_F$ inférieure à 100°C à une température $T_{HC}$ telle que $T_{HC}$ est supérieure à $T_F$, pour obtenir une phase grasse PG1 à l'état liquide ;
   2) dans une deuxième étape, à incorporer à la phase grasse PG1 à l'état liquide au moins une substance d'intérêt lipophile $S_L$ ;
   3) dans une troisième étape, à mettre en contact ladite phase grasse PG1 à l'état liquide avec une phase aqueuse PA préalablement portée à une température $T_{PA}$ telle que $T_{PA}$ est supérieure à $T_F$, ladite phase aqueuse renfermant en outre au moins une substance d'intérêt hydrophile $S_H$ et des particules solides colloïdales ;
   4) dans une quatrième étape, et toujours à une température supérieure à $T_F$, à soumettre le mélange liquide résultant de la troisième étape à une agitation mécanique pour obtenir une émulsion huile-dans-eau H/E formée de gouttelettes de phase grasse PG1 à l'état liquide dispersées dans la phase aqueuse continue et dans laquelle les particules solides colloïdales sont présentes à l'interface formée entre la phase aqueuse continue et les gouttelettes de phase grasse PG1 dispersées ;
   5) dans une cinquième étape, et toujours à une température supérieure à $T_F$, à ajuster le pH de l'émulsion H/E obtenue ci-dessus à l'étape précédente à une valeur inférieure ou égale à 1 à l'aide d'un agent acidifiant ;
   6) dans une sixième étape, et toujours à une température supérieure à $T_F$, à ajouter l'émulsion H/E obtenue ci-dessus à l'étape précédente, à une phase grasse PG2 comprenant une huile liquide à la température $T_{H/E}$ et renfermant en outre au moins un agent tensioactif non ionique et au moins un précurseur d'oxyde de silicium ;
   7) dans une septième étape, et toujours à une température supérieure à $T_F$, à soumettre le mélange résultant de la sixième étape à une agitation mécanique pour obtenir une émulsion double huile-dans-eau-dans-huile H/E/H formée d'une phase huileuse continue PG2 renfermant des gouttelettes de phase aqueuse PA, chacune desdites gouttelettes de phase aqueuse renfermant au moins une gouttelette de phase grasse PG1 à l'état solide ;
   8) dans une huitième étape, à laisser revenir l'émulsion double H/E/H obtenue ci-dessus à l'étape précédente jusqu'à une température $T_{H/E/H}$ inférieure à $T_F$, sans agitation, afin de provoquer d'une part la solidification de la phase grasse PG1, et la formation d'une enveloppe d'oxyde de silicium autour desdites gouttelettes de phase aqueuse PA et ainsi obtenir le matériau attendu ;
   9) dans une neuvième étape, à séparer ledit matériau de la phase grasse PG2.

2. Procédé selon la revendication 1, **caractérisé en ce que** les particules solides colloïdales sont des particules minérales choisies dans le groupe des oxydes, hydroxydes et sulfates de métaux.

3. Procédé selon la revendication 2, **caractérisé en ce que** les oxyde de métaux sont choisis parmi les oxydes de silicium, de titane, de zirconium et de fer.

4. Procédé selon la revendication 3, **caractérisé en ce que** les particules solides colloïdales sont choisies parmi les nanoparticules d'oxyde de silicium.

5. Procédé selon l'une quelconque des revendications 1 à 4, **caractérisé en ce que** la quantité de particules solides colloïdales varie de 0,01 % à 10% en masse par rapport à la masse totale de la phase aqueuse PA.

6. Procédé selon l'une quelconque des revendications 1 à 5, **caractérisé en ce que** les particules solides colloïdales sont fonctionnalisées par adsorption de molécules de tensioactif à leur surface.

7. Procédé selon l'une quelconque des revendications 1 à 6, **caractérisé en ce que** la quantité de phase grasse PG1 utilisée lors de la troisième représente au plus 40 % en masse par rapport à la masse de la phase aqueuse.

8. Procédé selon l'une quelconque des revendications 1 à 7, **caractérisé en ce que** pH de la phase aqueuse lors de la cinquième étape est ajusté à une valeur variant de -0,5 à 0,5.

9. Procédé selon l'une quelconque des revendications 1 à 8, **caractérisé en ce que** l'huile liquide à la température $T_{H/E}$ utilisée à titre de phase grasse PG2 lors de la sixième étape est être choisie parmi le polydiméthylsiloxane, l'huile d'arachide, l'huile de tournesol, les triesters, et leurs mélanges.

10. Procédé selon l'une quelconque des revendications 1 à 9, **caractérisé en ce que** le tensioactif nonionique est choisi parmi les polydiméthylsiloxanes cycliques, les copolymères blocs éthoxylés, les nonylphénols, et leurs mélanges.

11. Procédé selon l'une quelconque des revendications 1 à 10, **caractérisé en ce que** le tensioactif nonionique représente de 1 à 5% en masse par rapport à la masse totale de la phase grasse PG2.

12. Procédé selon l'une quelconque des revendications 1 à 11, **caractérisé en ce que** lors de la sixième étape, l'émulsion H/E représente au maximum 20 % en masse de la masse de la phase grasse PG2.

13. Procédé selon l'une quelconque des revendications 1 à 12, **caractérisé en ce que** les précurseurs d'oxyde de silicium sont choisis parmi les alcoxydes de silicium.

14. Procédé selon la revendication 13, **caractérisé en ce que** les alcoxydes de silicium sont choisis parmi le tetraméthoxyorthosilane, le tetraéthoxyorthosilane, le diméthyldiéthoxysilane, le (3-mercaptopropyl)trimethoxysilane, le (3-aminopropyl)triéthoxysilane, le N-(3-trimethoxysilylpropyl)pyrrole, le 3-(2,4-dinitrophénylamino)-propyltriéthoxysilane, le N-(2-aminoéthyl)-3-aminopropyltrimethoxysilane, le phényltriéthoxysilane, le méthyltriéthoxysilane et leurs mélanges.

15. Procédé selon l'une quelconque des revendications 1 à 14, **caractérisé en ce que** la quantité de précurseur d'oxyde de silicium varie de 1 à 7% en masse par rapport à la masse totale de l'émulsion double.

**Patentansprüche**

1. Verfahren zur Herstellung eines Materials in Form von festen Partikeln mit einem Durchmesser, der von 1 $\mu$m bis 1 cm variiert, bestehend aus einem kontinuierlichen Mantel, der mindestens ein Siliciumoxid umfasst, wobei der Mantel eine wässrige Phase einschließt, wobei die wässrige Phase mindestens eine bestimmte hydrophile Substanz $S_H$ enthält, und mindestens einen Tropfen einer fetten Phase, umfassend von 50 bis 99,9 Massenprozent mit Bezug auf die Masse der fetten Phase eines kristallisierbaren Öls in festem Zustand bei einer Temperatur von weniger als oder gleich 20 °C, wobei das kristallisierbare Öl eine Fusionstemperatur $T_F$ von weniger als 100 °C aufweist und mindestens eine bestimmte liophile Substanz $S_L$ enthält, **dadurch gekennzeichnet, dass** das Verfahren die folgenden Schritte umfasst:

1) in einem ersten Schritt, Bringen einer ersten fetten Phase PG1, umfassend von 50 bis 99,9 Massenprozent mit Bezug auf die Masse der fetten Phase PG1 eines festen kristallisierbaren Öls HC, das eine Fusionstemperatur $T_F$ aufweist, die niedriger als 100 °C ist, auf eine Temperatur $T_{HC}$, so dass $T_{HC}$ höher als $T_F$ ist, um eine fette Phase PG1 in flüssigem Zustand zu erhalten;
2) in einem zweiten Schritt, Einschließen, in die fette Phase PG1 in flüssigem Zustand, mindestens einer bestimmten liophilen Substanz $S_L$;
3) in einem dritten Schritt, In-Kontakt-Bringen der fetten Phase PG1 in flüssigem Zustand mit einer wässrigen Phase PA, die zuvor auf eine Temperatur $T_{PA}$ gebracht wurde, so dass $T_{PA}$ höher als $T_F$ ist, wobei die wässrige Phase außerdem mindestens eine bestimmte hydrophile Substanz $S_H$ und kolloidale feste Partikel enthält;
4) in einem vierten Schritt, und immer noch bei einer Temperatur höher als $T_F$, Unterziehen der flüssigen Mischung, die sich aus dem dritten Schritt ergibt, einem mechanischen Rühren, um eine Emulsion Öl-in-Wasser H/E zu erhalten, gebildet aus Tröpfchen der fetten Phase PG1 in flüssigem Zustand, dispergiert in der kontinuierlichen wässrigen Phase, und wobei die kolloidalen festen Partikel in der Schnittstelle vorhanden sind, die zwischen der kontinuierlichen wässrigen Phase und den dispergierten Tröpfchen der fetten Phase PG1 gebildet ist;

5) in einem fünften Schritt, und immer noch bei einer Temperatur höher als $T_F$, Anpassen des pH der Emulsion H/E, erhalten oben im vorhergehenden Schritt, an einen Wert von weniger als oder gleich wie 1 mit Hilfe eines Säuerungsmittels;

6) in einem sechsten Schritt, und immer noch bei einer Temperatur höher als $T_F$, Zugeben der Emulsion H/E, erhalten oben im vorhergehenden Schritt, zu einer fetten Phase PG2, umfassend ein flüssiges Öl mit der Temperatur $T_{H/E}$ und enthaltend außerdem mindestens ein nicht ionisches oberflächenaktives Mittel und mindestens einen Vorläufer von Siliciumoxid;

7) in einem siebten Schritt, und immer noch bei einer Temperatur höher als $T_F$, Unterziehen der Mischung, die sich aus dem sechsten Schritt ergibt, einem mechanischen Rühren, um eine doppelte Emulsion Öl-in-Wasser-in-Öl H/E/H zu erhalten, gebildet aus einer kontinuierlichen ölhaltigen Phase PG2, enthaltend Tröpfchen der wässrigen Phase PA, wobei jedes der Tröpfchen der wässrigen Phase mindestens ein Tröpfchen der fetten Phase PG1 in festem Zustand enthält;

8) in einem achten Schritt, Zulassen, dass die doppelte Emulsion H/E/H, erhalten oben im vorhergehenden Schritt, zu einer Temperatur $T_{H/E/H}$ geringer als $T_F$, ohne Rühren zurückkehrt, um einerseits die Verfestigung der fetten Phase PG1 und die Bildung eines Mantels aus Siliciumoxid um die Tröpfchen der wässrigen Phase PA hervorzurufen und somit das gewünschte Material zu erhalten;

9) in einem neunten Schritt, Trennen des Materials von der fetten Phase PG2.

2. Verfahren nach Anspruch 1, **dadurch gekennzeichnet, dass** die kolloidalen festen Partikel Mineralpartikel sind, ausgewählt aus der Gruppe der Metalloxide, Metallhydroxide und Metallsulfate.

3. Verfahren nach Anspruch 2, **dadurch gekennzeichnet, dass** die Metalloxide ausgewählt sind aus den Silicium-, Titan-, Zirkonium- und Eisenoxiden.

4. Verfahren nach Anspruch 3, **dadurch gekennzeichnet, dass** die kolloidalen festen Partikel ausgewählt sind aus den Siliciumoxid-Nanopartikeln.

5. Verfahren nach einem der Ansprüche 1 bis 4, **dadurch gekennzeichnet, dass** die Menge von kolloidalen festen Partikeln von 0,01 Massenprozent bis 10 Massenprozent mit Bezug auf die Gesamtmasse der wässrigen Phase PA variiert.

6. Verfahren nach einem der Ansprüche 1 bis 5, **dadurch gekennzeichnet, dass** die kolloidalen festen Partikel durch Adsorption von Molekülen eines Tensids an ihrer Oberfläche funktionalisiert sind.

7. Verfahren nach einem der Ansprüche 1 bis 6, **dadurch gekennzeichnet, dass** die Menge von fetter Phase PG1, verwendet beim dritten Schritt, höchstens 40 Massenprozent mit Bezug auf die Masse der wässrigen Phase darstellt.

8. Verfahren nach einem der Ansprüche 1 bis 7, **dadurch gekennzeichnet, dass** der pH-Wert der wässrigen Phase beim fünften Schritt auf einen Wert eingestellt ist, der von -0,5 bis 0,5 variiert.

9. Verfahren nach einem der Ansprüche 1 bis 8, **dadurch gekennzeichnet, dass** das flüssige Öl bei der Temperatur $T_{H/E}$, verwendet als fette Phase PG2 beim sechsten Schritt, ausgewählt ist aus Polydimethylsiloxan, Erdnussöl, Sonnenblumenöl, Triestern und ihren Mischungen.

10. Verfahren nach einem der Ansprüche 1 bis 9, **dadurch gekennzeichnet, dass** das nicht ionische Tensid ausgewählt ist aus cyclischen Polydimethylsiloxanen, ethoxylierten Copolymerblöcken, Nonylphenolen und ihren Mischungen.

11. Verfahren nach einem der Ansprüche 1 bis 10, **dadurch gekennzeichnet, dass** das nicht ionische Tensid 1 bis 5 Massenprozent mit Bezug auf die Gesamtmasse der fetten Phase PG2 darstellt.

12. Verfahren nach einem der Ansprüche 1 bis 11, **dadurch gekennzeichnet, dass** beim sechsten Schritt die Emulsion H/E maximal 20 Massenprozent der Masse der fetten Phase PG2 darstellt.

13. Verfahren nach einem der Ansprüche 1 bis 12, **dadurch gekennzeichnet, dass** die Vorläufer von Siliciumoxid ausgewählt sind aus den Siliciumalcoxyden.

14. Verfahren nach Anspruch 13, **dadurch gekennzeichnet, dass** die Siliciumalcoxyde ausgewählt sind aus Tetramethoxyorthosilan, Tetraethoxyorthosilan, Dimethyldiethoxysilan, (3-Mercaptopropyl)trimethoxysilan, (3-Aminopropyl)triethoxysilan, N-(3-Trimethoxysilylpropyl)pyrrol, 3-(2,4-Dinitrophenylamino)-propyltriethoxysilan, N-(2-Aminoethyl)-3-aminopropyltrimethoxysilan, Phenyltriethoxysilan, Methyltriethoxysilan und ihren Mischungen.

15. Verfahren nach einem der Ansprüche 1 bis 14, **dadurch gekennzeichnet, dass** die Menge von Siliciumoxidvorläufer von 1 bis 7 Massenprozent mit Bezug auf die Gesamtmasse der doppelten Emulsion variiert.

**Claims**

1. Method for preparing a material in the form of solid particles having a diameter varying between 1 μm and 1 cm formed by a continuous shell comprising at least one silicon oxide, said shell enclosing an aqueous phase, said aqueous phase containing at least one hydrophilic substance of interest $S_H$ and at least one droplet of a fatty phase comprising from 50 to 99.9% by weight in relation to the weight of said fatty phase of crystallisable oil in the solid state at a temperature less than or equal to 20°C, said crystallisable oil having a melting temperature $T_F$ less than 100°C, and containing at least one lipophilic substance of interest $S_L$, **characterised in that** said method comprises the following steps consisting:

    1) in a first step, in bringing a first fatty phase PG1 comprising from 50 to 99.9% by weight in relation to the weight of said fatty phase PG1 of a solid crystallisable oil HC having a melting temperature $T_{HC}$ such that $T_{HC}$ is greater than $T_F$, in order to obtain a fatty phase PG1 in the liquid state;
    2) in a second step, in incorporating at the fatty phase PG1 in the liquid state at least one lipophilic substance of interest $S_L$;
    3) in a third step, in placing in contact said fatty phase PG1 in the liquid state with an aqueous phase PA brought beforehand to a temperature $T_{PA}$ such that $T_{PA}$ is greater than $T_F$, said aqueous phase containing furthermore at least one hydrophilic substance of interest $S_H$ and solid colloidal particles;
    4) in a fourth step, and still at a temperature greater than $T_F$, in subjecting the liquid mixture resulting from the third step to a mechanical stirring in order to obtain an oil-in-water emulsion H/E formed from droplets of fatty phase PG1 in the liquid state dispersed in the continuous aqueous phase and wherein the solid colloidal particles are present at the interface formed between the continuous aqueous phase and the droplets of fatty phase PG1 dispersed;
    5) in a fifth step, and still at a temperature greater than $T_F$, in adjusting the pH of the H/E emulsion obtained hereinabove in the preceding step to a value less than or equal to 1 using an acidifying agent;
    6) in a sixth step, and still at a temperature greater than $T_F$, in adding the H/E emulsion obtained hereinabove in the preceding step, to a fatty phase PG2 comprising a liquid oil at the temperature $T_{H/E}$ and further containing at least one non-ionic surfactant and at least one silicon oxide precursor;
    7) in a seventh step, and still at a temperature greater than $T_F$, in subjecting the mixture resulting from the sixth step to a mechanical stirring in order to obtain a double oil-in-water-in-oil H/E/H emulsion formed from a continuous oily phase PG2 containing droplets of aqueous phase PA, each one of said droplets of aqueous phase containing at least one droplet of fatty phase PG1 in the solid state;
    8) in an eighth step, in allowing the double H/E/H emulsion obtained hereinabove in the preceding step to return to a temperature $T_{H/E/H}$ less than $T_F$, without stirring, in order to provoke on the one hand the solidification of the fatty phase PG1, and the formation of a shell of silicon oxide around said droplets of aqueous phase PA and as such obtain the expected material;
    9) in a ninth step, in separating said material from the fatty phase PG2.

2. Method according to claim 1, **characterised in that** the solid colloidal particles are mineral particles chosen from the group of metal oxides, hydroxides and sulphates.

3. Method according to claim 2, **characterised in that** the metal oxides are chosen from oxides of silicon, titanium, zirconium and iron.

4. Method according to claim 3, **characterised in that** the solid colloidal particles are chosen from nanoparticles of silicon oxide.

5. Method according to any of claims 1 to 4, **characterised in that** the quantity of solid colloidal particles varies from 0.01% to 10% by weight in relation to the total weight of the aqueous phase PA.

6. Method according to any of claims 1 to 5, **characterised in that** the solid colloidal particles are functionalised by adsorption of surfactant molecules at the surface thereof.

7. Method according to any of claims 1 to 6, **characterised in that** the quantity of fatty phase PG1 used during the third represents at most 40% by weight in relation to the weight of the aqueous phase.

8. Method according to any of claims 1 to 7, **characterised in that** the pH of the aqueous phase during the fifth step is adjusted to a value varying from -0.5 to 0.5.

9. Method according to any of claims 1 to 8, **characterised in that** the liquid oil at the temperature $T_{H/E}$ used in terms of the fatty phase PG2 during the sixth step is to be chosen from polydimethylsiloxane, groundnut oil, sunflower oil, triesters, and mixtures thereof.

10. Method according to any of claims 1 to 9, **characterised in that** the non-ionic surfactant is chosen from cyclic polydimethylsiloxanes, ethoxylated block copolymers, nonylphenols, and mixtures thereof.

11. Method according to any of claims 1 to 10, **characterised in that** the non-ionic surfactant represents from 1 to 5% by weight in relation to the total weight of the fatty phase PG2.

12. Method according to any of claims 1 to 11, **characterised in that** during the sixth step, the H/E emulsion represents at most 20% by weight of the weight of the fatty phase PG2.

13. Method according to any of claims 1 to 12, **characterised in that** the silicon oxide precursors are chosen from silicon alcoxides.

14. Method according to claim 13, **characterised in that** the silicon alcoxides are chosen from tetramethoxyorthosilane, tetraethoxyorthosilane, dimethyldiethoxysilane, (3-mercaptopropyl)trimethoxysilane, (3-aminopropyl)triethoxysilane, N-(3-trimethoxysilylpropyl)pyrrole, 3-(2,4-dinitrophenylamino)-propyltriethoxysilane, N-(2-aminoethyl)-3-aminopropyltrimethoxysilane, phenyltriethoxysilane, methyltriethoxysilane and mixtures thereof.

15. Method according to any of claims 1 to 14, **characterised in that** the quantity of silicon oxide precursor varies from 1 to 7% by weight in relation to the total weight of the double emulsion.

Figure 1

**Figure 2**

**RÉFÉRENCES CITÉES DANS LA DESCRIPTION**

**Documents brevets cités dans la description**

- FR 2948581 A

- WO 2011012813 A1